# EUROPEAN PATENT APPLICATION

(11) **EP 1 512 407 A1**
(43) Date of publication of application: **09.03.2005**
(21) Application number: 03255473.5
(22) Date of filing: 02.09.2003
(51) Int. Cl.: A61K 35/78, A61P 9/00, A61P 9/10

(54) **Use of fermented glycine max (L.) extract in inhibiting 15-lipoxygenase**

(71) Applicant: Microbio Company, Ltd., Taipei 115, Taiwan (TW)
(72) Inventor: LU, William Kung-Ming, Taipei 115, Taiwan (CN)
(74) Representative: Sexton, Jane Helen

(57) **Abstract**

This invention relates to a use of a fermented *Glycine max* (L.) extract prepared by fermenting an aqueous *Glycine max* (L.) extract with at least one lactic acid bacteria together with at least one yeast, in inhibiting 15-lipoxygenase. In particular, the fermented *Glycine max* (L.) extract can be used in preventing and/or treating a disease in which 15-lipoxygenase inhibition is implicated in a subject, such as cardiovascular diseases, immune disorders(e.g., asthma and inflammation), and modulating the immune system. The invention also relates to a use of the fermented *Glycine max* (L.) extract in treating and/or preventing a microbe infection.

## Description

### TECHNICAL FIELD

This invention relates to a use of a fermented *Glycine max* (L.) extract in inhibiting 15-lipoxygenase, preventing and/or treating a disease in which 15-lipoxygenase inhibition is implicated in a subject, such as cardiovascular diseases, immune disorders (e.g., asthma and or inflammation), and modulating the immune system. The invention also relates to a use of the fermented *Glycine max* (L.) extract in preventing and/or treating a microbe infection.

### BACKGROUND OF THE INVENTION

Lipoxygenases (LOX) are nonheme iron-containing enzyme that catalyze the oxygenation of certain polyunsaturated fatty acids such as lipoproteins. Several different lipoxygenase enzymes, e.g. LOX-5, LOX-12 and LOX-15, are known, each having a characteristic oxidation action. LOX-15 catalyzes the oxygenation of arachidonic and linoleic acids and has been implicated in the oxidative modification of low-density lipoproteins (LDL). Many researches reported that the LOX-15 is associated with coronary artery disease and atherosclerosis (Shen *et al.,* J. Clin. Invest. 1996, Vol. 98, No. 10, pp. 2201-2208; Timo *et al.,* 1995, Vol. 92 (11), pp. 3297-3303; *Ravalli et al.,* 1995, Arteriosclerosis, Thrombosis and Vascular Biology, Vol. 15, No. 3, pp. 340-348; and Kuhn *et al.,* 1997, J. Clin. Invest., Vol. 99, No. 5, pp. 888-893), cancer and inflammatory diseases (USP 6,001,866; *Mogul et al.,* 2001, Biochemistry, 40, 4391-4397; Walther *et al.,* 1999, Molecular Pharmacology, 56: 196-203; and Kamitani *et al.,* 1998, the Journal of Biological Chemistry, Vol. 273, No. 34, pp. 21569-21577 ), and immune response (Kruisselbrink *et al.,* 2001, Clin Exp Immunol, 126:2-8). Therefore, a substance having an efficacy in inhibiting LOX is useful as an agent for preventing or treating diseases associated with LOX.

Soybeans are one concentrated source of isoflavones in human diet. They also contain many compounds including saponins, phytosterols, soybean phytates, protease inhibitors, phenolic acids, complex sugars, boron, lecithin, omega-3 fatty acids and folic acid. They can impart health benefits. Many eastern traditional foods, such as tembe and natto, are produced from the fermentation of soybeans. For example, tembe is produced by fermenting soybean with *Rhizopus oligosporus, R. oryzae, R. arrihizus* and *R. stolonifer.* Natto is produced by fermenting soybean with *Bacillus natto.* The traditional fermented foods can be used as a superior protein origin. However, none of the prior art discloses that any known fermented soybean foods and soybeans have an efficacy in inhibiting 15-lipoxygenase LOX-15.

### SUMMARY OF THE INVENTION

This invention relates to a use of a fermented *Glycine max* (L.) extract, which is prepared by fermenting an aqueous *Glycine max* (L.) extract with at least one lactic acid bacteria together with at least one yeast, in inhibiting 15-lipoxygenase (LOX-15). Particularly, *Glycine max* (L.) is soybean or black soybean. More particularly, the fermented *Glycine max* (L.) extract of the invention include the fermented soybean extract and fermented black soybean extract.

The fermented *Glycine max* (L.) extract of the invention can be used in preventing and/or treating a disease in which 15-lipoxygenase inhibition is implicated in a subject, such as cardiovascular diseases, immune disorders (e.g., asthma and inflammation), and modulating the immune system.

The fermented *Glycine max* (L.) extract of the invention can also be used in preventing and/or treating a microbe infection in a subject.

### BRIEF DESCRIPTION OF THE DRAWINGS

Figure 1 shows the inhibition of LOX-15 with the fermented soybean extract.
Figure 2 shows the relation of the concentration of the black soybean and its inhibition rate to LOX-15.

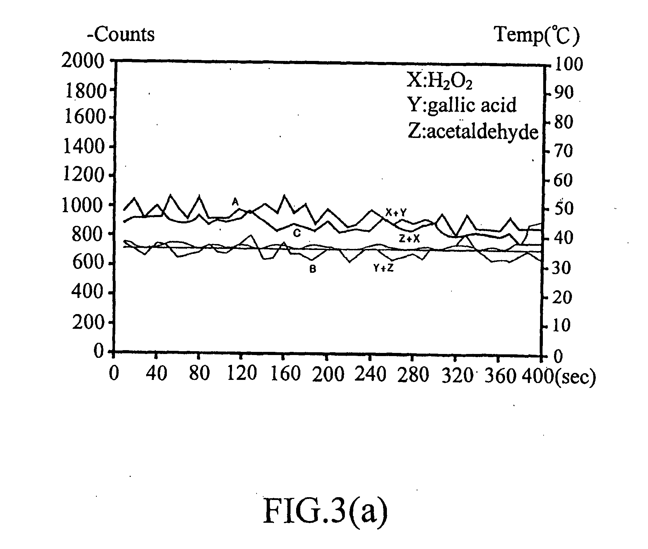

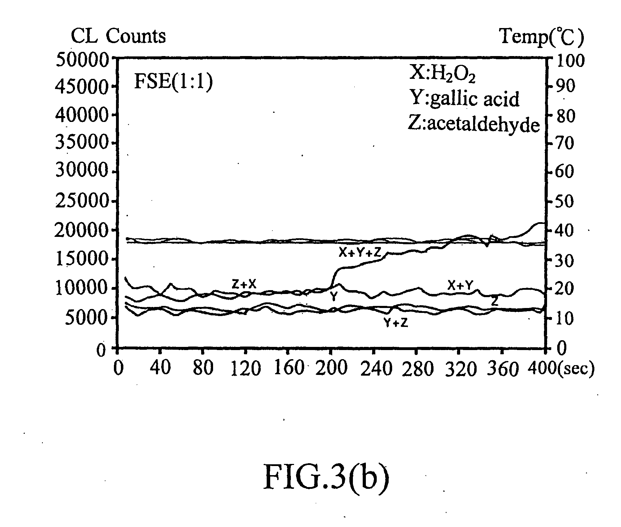

Figure 3 shows the antioxidant effect of gallic acid. At a temperature of 37°C, chemiluminescence, CL Counts, was measured after the addition of H₂O₂ as a peroxide (X), gallic acid as an antioxidant (Y) and/or acetaldehyde as a radical receptor (Z). In Figure 3(a), curve A refers to the chemiluminescence after the addition of X and Y together; curve B refers to the chemiluminescence after the addition of Y and Z together; and curve C refers to the chemiluminescence after the addition of X and Z together. Figure 3(b) shows that, when gallic acid was added into a mixture of X and Z at 200 seconds, the chemiluminescence was increased (i.e. the chemiluminescence occurred only in the presence of X, Y and Z all together).

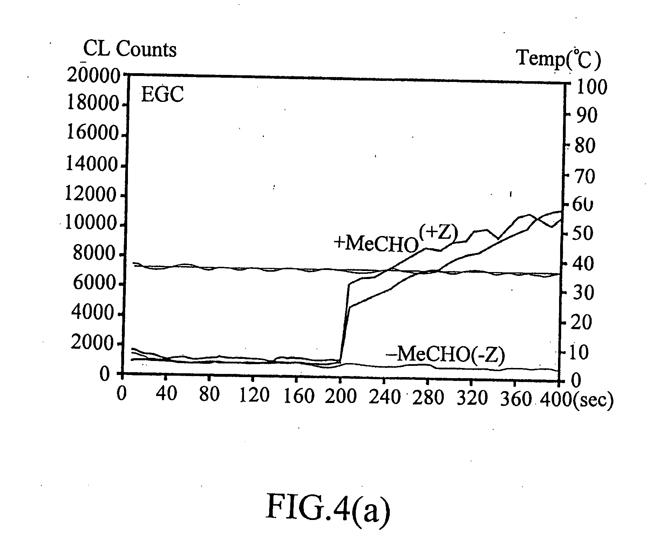

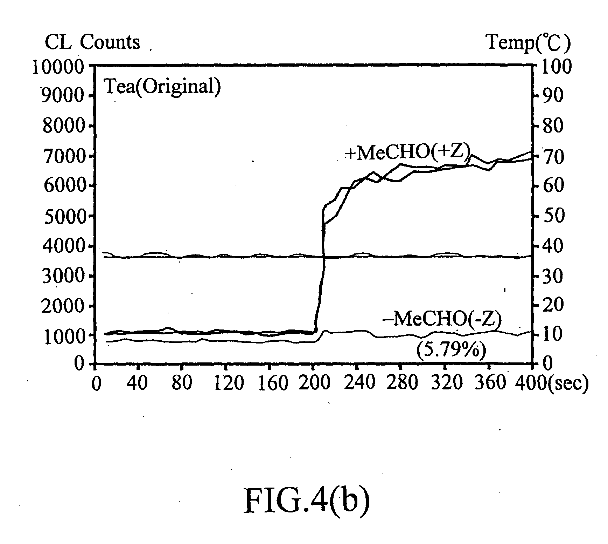

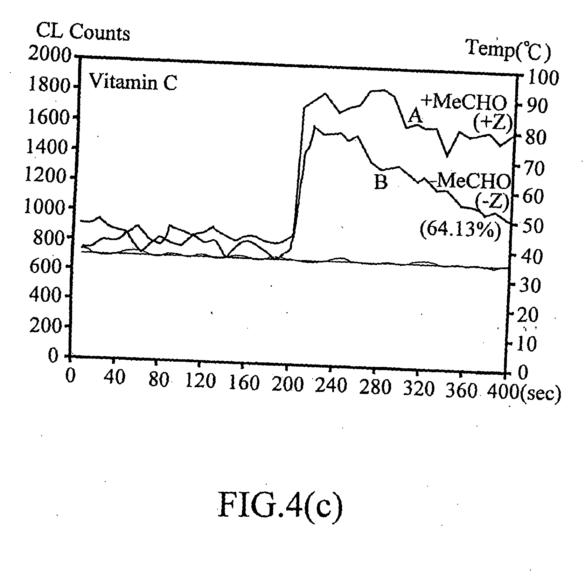

Figure 4 shows the antioxidant effects of tea and Vitamin C. At a temperature of 37°C, chemiluminescence, in CL Counts, was measured in the presence of H₂O₂ as a peroxide with the presence or absence acetaldehyde (Z) as a radical receptor, and an addition of one of several antioxidants, i.e. EGC, tea and vitamin C, at 200 seconds. Figure 4(a) shows the chemiluminescence determined with or without acetaldehyde when EGC, i.e. epigallocatechin which is a polyphenol, was added as the antioxidant at 200 seconds. Figure 4(b) shows that when tea was added as the antioxidant at 200 seconds, its chemiluminescence emitted in the absence of acetaldehyde (+Z) is 5.79% of that in the presence of acetaldehyde. Figure 4(c) shows that, when vitamin C was used as the antioxidant, the chemiluminescence intensity in the absence of acetaldehyde was 64.13% of that detected in the presence of acetaldehyde.

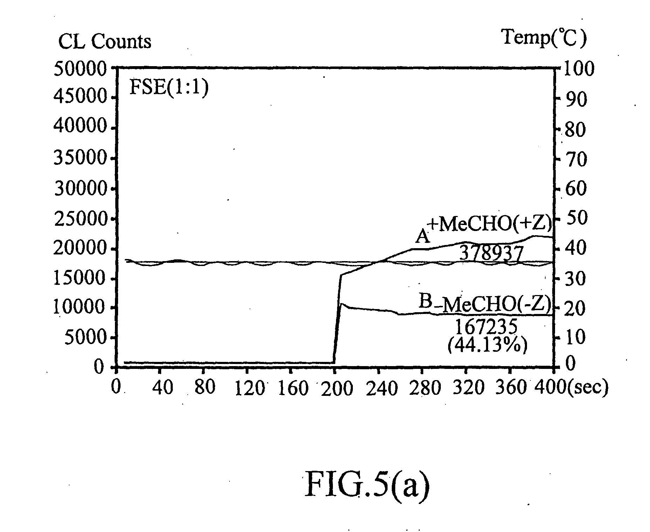

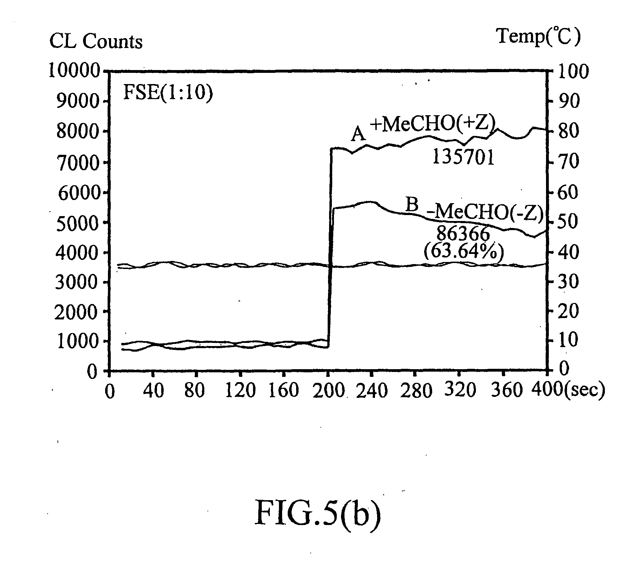

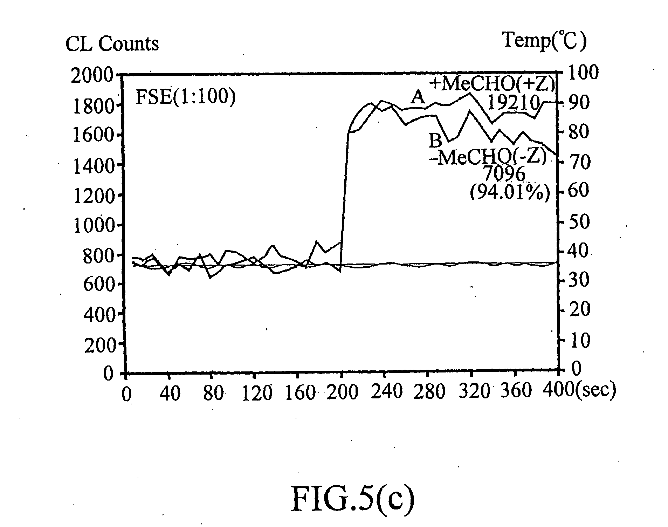

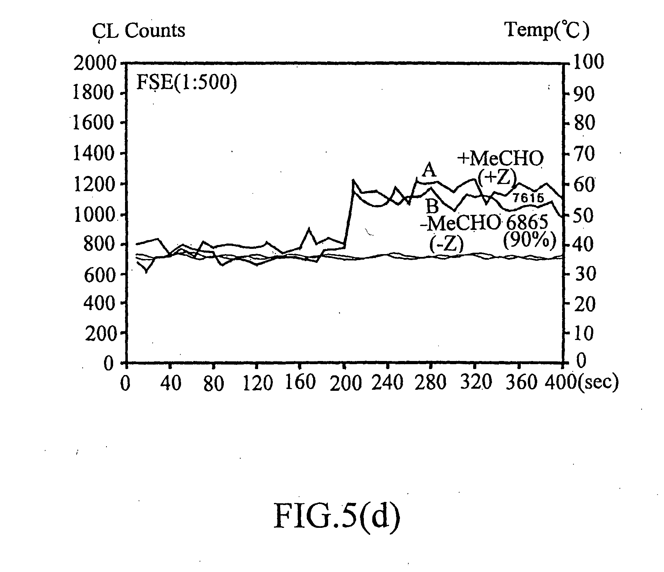

Figure 5 shows the antioxidant effect of different concentrations of the fermented soybean extract, FSE. At a temperature of 37°C, with H₂O₂ used as a peroxide in the presence or absence of acetaldehyde as a radical receptor (Z), the chemiluminescence was measured and the fermented soybean extract at different concentrations was added at 200 seconds. Figure 5(a) shows that, at a FSE concentration of 1:1, the chemiluminescence intensity in the absence of acetaldehyde was 44.13% of that in the presence of acetaldehyde. Figure 5(b) shows that, at a FSE concentration of 1:10, the chemiluminescence intensity in the absence of acetaldehyde was 63.64% of that in the presence of acetaldehyde. Figures 5(c) and (d) show that, at a FSE concentration of 1:100 or 1:500, the chemiluminescence intensity in the absence of acetaldehyde was at least 90% of that in the presence of acetaldehyde.
Figure 6 shows the effect of the fermented soybean extract on the total plasma cholesterol levels in apoE-deficient mice.
Figure 7 shows the effect of the fermented soybean extract on the lesion formation in apoE-deficient mice.
Figure 8 shows the staining of aortic lesions from apoE-deficient mice.

### DETAILED DESCRIPTION OF THE INVENTION

The invention relates to a new use of a fermented *Glycine max* (L.) extract for the production of a composition for use in the inhibition of 15-lipoxygenase (LOX-15) and the diseases implicated in the 15-lipoxygenase, wherein the fermented *Glycine max* (L.) extract is prepared by fermenting an aqueous *Glycine max* (L.) extract with at least one lactic acid bacteria together with at least one yeast. It is unexpectedly found that the fermented *Glycine max* (L.) extract can effectively inhibit 15-lipoxygenase in a low level.

In particular, the composition comprising the fermented *Glycine max* (L.) extract of the invention can be used in preventing and/or treating a disease in which 15-lipoxygenase inhibition is implicated in a subject, such as cardiovascular diseases, immune disorders (e.g., asthma and inflammation), and modulating the immune system. In addition, the fermented soybean extract of the invention can also be used in treating and/or preventing a microbe infection.

The composition of the present invention can be a pharmaceutical composition or a food composition.

### Process for Producing the Fermented Soybean Extract

The fermented *Glycine max* (L.) extract is made by fermentation of an aqueous *Glycine max* (L.) extract with at least one lactic acid bacteria together with at least one yeast, followed by sterilization, e.g. by heat, of the fermented liquid with optional filtration and concentration.

According to the invention, the preferred *Glycine max* (L.) used in the preparation of the fermented *Glycine max* (L.) extract is selected from the group consisting of soybean and black soybean. More particularly, the fermented *Glycine max* (L.) extract of the invention is the fermented soybean extract or fermented black soybean extract.

The fermented *Glycine max* (L.) extract is produced by fermentation of *Glycine max* (L.) extract with at least one lactic acid bacteria, e.g. one or more strains of a *Lactobacillus* species or several strains of a number of *Lactobacillus* species, together with at least one yeast, e.g. one or more strains of a *Saccharomyces* species or several strains of a number of *Saccharomyces* species. The fermentation of the aqueous *Glycine max* (L.) extract with one or more lactic acid bacteria and the optional *Saccharomyces* species can be carried out sequentially in any order or simultaneously, preferably simultaneously.

If more than one microbe is used in the fermentation, the fermentation can be conducted with the microbes sequentially or simultaneously. Preferably, an aqueous extract of non-genetically modified organic *Glycine max* (L.) of selected grade is used as a starting material. Preferably, the fermentation is carried out using a heterogeneous culture of *Lactobacillus,* for example, a culture of 5, 10, 15, 20, 25 or 30 strains of *Lactobacillus* and at least one yeast is added to the heterogeneous culture of *Lactobacillus.* The strains of *Lactobacillus* that can be used include, for examples, *Lactobacillus acidophilus* CCRC 10695, 14026, 14064, 14065 and/or 14079, *Lactobacillus delbrueckii bulgaricus* CCRC 10696, 14007, 14009, 14010, 14069, 14071, 14098 and/or 16054, *Lactobacillus lactis lactis* CCRC 10791, 12267, 12306, 12312, 12315, 12323, 14016, 14015 and/or 14117, *Lactobacillus kefir* CCRC 14011, and/or *Lactobacillus kefiranofaciens* CCRC 16059. The yeast that can be used include, for example, *Saccharomyces cerevisiae* CCRC 20577, 20578, 20581, 21494, 21550, 21797, 21805, 22138, 22234, 22337, 22731 and/or 22728, and/or *Candida kefyr* CCRC 21269, 21742 and/or 22057. After fermentation, the fermented liquid is sterilized, e.g. by heat or irradiation, preferably by heat, to obtain a sterilized liquid. Preferably, the sterilized liquid is filtered or centrifuged, preferably filtered, to remove most or all of the dead microbes to obtain the fermented *Glycine max* (L.) extract. More preferably, the filtration step is followed by removal of some of the water from the filtrate to concentrate the fermented liquid to obtain the fermented *Glycine max* (L.) extract. Unless otherwise specified, the tests performed in this application involved the fermented *Glycine max* (L.) extract after the concentration step. Optionally, the fermented *Glycine max* (L.) extract can be dried, e.g. via lyophilization, to obtain the fermented *Glycine max* (L.) extract in a powder form.

The process can be carried out by mixing organic *Glycine max* (L.) (with fat removed) with distilled water at a ratio of 1: 10. The mixture is heated at 100° C for 30 minutes and then filtered to obtain a *Glycine max* (L.) extract. Beef and kelp are boiled in distilled water for 30 minutes to obtain a broth. Salt, sugar and agar are added to produce a special agar medium. The lactic acid bacteria and yeast strains are added to the special agar medium. The lactic acid bacteria with the optional inclusion of the yeast in the medium are transferred to the *Glycine max* (L.) extract and incubated at 36-43° C for 45-50 hours. Preferably, the various strains of the microbes are grouped according to similar growth characteristics, e.g. any requirements of unique nutrient medium, whether the microbial strains could produce a good smell after fermentation and whether the grouped microbes can survive in the unique condition, so that groups of the microbes are added to the *Glycine max* (L.) extract separately before the incubation. The purpose of this step is to reduce any negative interaction among the various strains. Also preferably, equal proportion of the different groups of microbial strains are added to the *Glycine max* (L.) extract before the incubation and the resulting extract is incubated at 40° C for 45-47 hours. Upon completion of the incubation period, the heterogeneous culture is then transferred to the *Glycine max* (L.) extract again and incubated at 36-43° C for 100-150 hours. The final fermented extract is heat sterilized and filtered; and 95% of the water content of the filtrate is removed in a concentrator to obtain a fermented *Glycine max* (L.) extract in a concentrated or condensed form. The upper layer is then filtered through porcelain, and thereafter dispensed in containers and sealed.

Within the scope of the present invention is a fermented extract of a Chinese herb prepared in a process similar to the one described above with the substitution of the soybean with the Chinese herb. The fermented extract of the Chinese herbs can be *Glycyrrhiza uralensis Fish, Lycium barbarum, Coix lacryma-jobi L var., ma-yune Stapf, Sophora tonkinensis gapnep., Cassia btusifolia., Scutellaria baicalensis Georgi, Artemisia capillaries Thunb., Coptis chinensis Frsnch., Gentiana scabra Bge., Nelumbo nucifera Gaertn., Chrysantheiferamum morifolium Ramat., Gardenia jasminoides Ellis, Hordeum vulgare L., Cinnamomum cassia Presl, Raph, anus sativus L., Dioscorea opposita Thunb., Angelica sinensis (Oliv.), Ligusticum chuanxiong Hort., Notopterygium incisum, Paeonia lactiflora Pall., Allium satium L., Schisandra chinensis(Turcz.)Baill, Rehmannia glutinosa Libosch., Acanthopanax gracilistylus W.W.Smith, Equus asinus L*., *Ligustrum lucidum Ait., Phaseolus radiatus L., Triticum aestivum L., Dolichos lablab L., Atractylodes macrocephala Koidz., Saposhnikovia divaricata, Lonicera japonica Thund., Cinnamomum cassia Presl, Zingiber officinale Rosc., Gastrodia elata Bl., Asparagus cochinchinensis(Liur.)Merr., Dendrobiun loddigesii Rolfe., and Sesamum indicum L.*

### Use in the Inhibition of 15-lipoxyenase

15-lipoxygenases are nonheme iron-containing enzymes that catalyze the oxygenation of certain polyunsaturated fatty acids such as lipoproteins. It is known that compounds inhibit the action of 15-lipoxygenase enzyme are useful in the treatment or alleviation of inflammatory diseases, allergy, cardiovascular diseases and immune disorders in mammals including humans.

Studies in the invention have demonstrated that the fermented *Glycine max* (L.) extract of the present invention can inhibit 15-lipoxygenases superior than the known fermented soybean foods and the unfermented soybean.

### Use as an Antioxidant

The fermented *Glycine max* (L.) extract has prominent antioxidant and free radical scavenger activities. The fermented *Glycine max* (L.) extract can remove superoxide free radicals, e.g., O₂⁻, H₂O₂ and ROO⁻, and can act as an antioxidant for unsaturated fatty acid and fat. The fermented *Glycine max* (L.) extract has a prominent ability to eliminate hyper oxygen anions to protect the cell from oxidative injury and change free radicals to harmless substances with an energy decreasing procedure.

### Use as Agent against Cardiovascular Diseases

Many studies shows that the inhibitors of LOX-15 in the treatment and prevention of inflammation and atherosclerosis (Cornicelli *et al.,* USP 6,001,866; Bocan *et al.,* Atherosclerosis, 136, 203-216, 1998 and Timo *et al.,* Circulation, col. 92 (1), 1 December 1995, pp. 3297-3303). It is expected that the fermented *Glycine max* (L.) extract is useful for preventing and/or treating cardiovascular diseases, such as atherosclearosis.

### Use for Promoting Immune Function

*In vitro* study indicated that the fermented *Glycine max* (L.) extract of the invention improved immune function. The effect of the fermented *Glycine max* (L.) extract on modulation of the immunity of animals (Bala/c mice) was studied by treating the animal with the fermented *Glycine max* (L.) extract combined with or without a challenge with various mitogens including lipopolysachrride, concanavalin A and phytohaemagglutilin. Spleen cell proliferation assay indicated that the fermented *Glycine max* (L.) extract could be related with T & B cell interaction in immunity modulation. The fermented *Glycine max* (L.) extract can also be correlated with anti-inflammation reaction. The *Glycine max* (L.) extract also enhanced phagocytosis activity of macrophages by 71%. Similar results were found with in vivo studies in mice. It was also demonstrated that the anti-tumor effect of fermented *Glycine max* (L.) extract is mediated by cytokines released. Conditioned medium from fermented *Glycine max* (L.) extract-stimulated peripheral blood mononuclear cells by 45-56%. Levels of interleukin-1b, interleukin-b and tumor necrosis factor-a were much higher than those of untreated control. Since untreated Macrophages and T Lymphocytes produced little or no cytokine and normal mononuclear cells did not suppress leukemic cell growth, the anti-tumor activity is speculated to be derived from elevated level of cytokine.

The fermented *Glycine max* (L.) extract is beneficial to asthmatic children. For example, results obtained also showed significant body weight gain in a group of children with asthma when administered with 3 ml of the fermented soybean extract daily for 4 months. Blood tests showed that taking fermented *Glycine max* (L.) extract increase the RBC and Hb levels in these asthmatic children.

### Use as Anti-Inflammation Agent

The invention also relates to a method for treating and/or preventing inflammation in a subject, comprising administering an effective amount of a fermented *Glycine max* (L.) extract to the subject in need of thereof, wherein the fermented *Glycine max* (L.) extract is prepared by fermenting an aqueous *Glycine max* (L.) extract with at least one lactic acid bacteria together with at least one yeast.

The fermented *Glycine max* (L.) extract has demonstrated anti-inflammatory effect at dosage of 10ml/kg on the reduction of carrageenan induced hind paw edema in rats and anti-inflammatory effect on acute and chronic arthritis in adjuvant arthritis test.

### Use for Preventing or Treating Infections

The fermented *Glycine max* (L.) extract has demonstrated antimicrobial activity *in vitro* and *in vivo.* It inhibits the growth of *Helicobacter pylori,* ampicillin and methycillin resistant *Staphylococcus aureus, Salmonella typhimurium, Bacillus subtilis, E.coli, Proteus vulgaris* and Vancomycin resistant *Enterococci.* Preferably, the Vancomycin resistant *Enterococcus* is selected from the group consisting of *E. avium, E. casseliflavus, E. durans, E faecalis* and *E. faecium.* More Preferably, the Vancomycin resistant *Enterococcus* is selected from the group consisting of *E. avium, E. casseliflavus, E. durans* and *E .faecali.* More Preferably, the Vancomycin resistant *Enterococcus* is selected from the group consisting of *E. durans, E. faecalis* and *E. faecium.* More Preferably, the Vancomycin resistant *Enterococcus* is selected from the group consisting of *E. avium, E .faecalis* and *E. faecium.* More Preferably, the Vancomycin resistant *Enterococcus* is selected from the group consisting of *E .faecalis* and *E. faecium.* Most preferably, the Vancomycin resistant *Enterococcus* is *E .faecalis.*

The effective concentration of fermented *Glycine max* (L.) extract is generally in the range of 1- 10%. The selective antimicrobial decontamination effect of fermented *Glycine* *max* (L.) extract for prophylaxis of bacterial infection in patients who are under risk of developing neutropenia due to the concurrent treatment of anti-cancer chemotherapy is also demonstrated in 100 patients. Preferably, the vancomycin resistant *Enterococcus* is selected from the group consisting of *E. avium, E. casseliflavus, E. durans, E .faecalis* and *E. faecium.* More Preferably, the vancomycin resistant *Enterococcus* is selected from the group consisting of *E. avium, E. casseliflavus, E. durans and E .faecali.* More Preferably, the vancomycin resistant *Enterococcus* is selected from the group consisting of *E. durans, E. faecalis* and *E. faecium.* More Preferably, the vancomycin resistant *Enterococcus* is selected from the group consisting of *E. avium, E faecalis* and *E. faecium.* More Preferably, the vancomycin resistant *Enterococcus* is selected from the group consisting of *E .faecalis* and *E. faecium.* Most preferably, the vancomycin resistant *Enterococcus* is *E .faecalis.*

### Administration of the Fermented Glycine max (L.) Extract

In this invention, the fermented *Glycine max* (L.) extract may be administered alone or in a composition comprising the fermented *Glycine max* (L.) extract and a pharmaceutically acceptable carrier, diluent and/or excipient. Preferably, the fermented *Glycine max* (L.) extract is fermented soybean extract or fermented black soybean extract. The fermented *Glycine max* (L.) extract may be administered at a dose of about 0.001 to 40 ml/kg body weight, with a maximum dose of 2000 ml per person per administration. Preferably, the dose of the fermented *Glycine max* (L.) extract is 0.01 to 20 ml/kg, more preferably 0.1 to 5 ml/kg, body weight of the subject. These doses are based on the fermented *Glycine max* (L.) extract in the concentrated form, but appropriate doses of the fermented *Glycine max* (L.) extract in the unconcentrated form or dry powder form can be calculated accordingly. The dose can be adjusted based on the health condition of the subject or the disease to be prevented or treated.

The fermented *Glycine max* (L.) extract was demonstrated to be highly safe for daily intake of 1-10 ml on a long-term basis in a 6 months chronic toxicity study of rodents. Mice receiving a dose of 10 ml/kg and 1 ml/kg for 28 days did not exhibit any significant difference or abnormal symptom in a subacute oral toxicity study. No signs of gross toxicity or mortality were observed in two groups of tested animals administered 20 ml/kg and 1 ml/kg in an acute oral toxicity study of rodents. The fermented *Glycine max* (L.) extract was demonstrated to be non-mutagenic in Ames test, to not cause chromosomal damage in mammalian cells in vitro and to not induce micronuclei in bone marrow cells in ICR mice tested.

When the fermented *Glycine max* (L.) extract is administered in pregnant women, the dosage of the fermented *Glycine max* (L.) extract can be increased during pregnancy until the daily intake reaches 12 ml. The fermented *Glycine max* (L.) extract can be administered at early and midstage pregnancy, as well as delivery. The results showed that the fermented *Glycine max* (L.) extract could improve symptoms, including constipation, nausea, vomiting, and gastrointestinal discomfort, commonly found in pregnancy. In addition, the administration of the fermented *Glycine max* (L.) extract can reduce abnormalities during pregnancy and at delivery. The fermented *Glycine max* (L.) extract is not only good for health improvement during pregnancy, but it also produces no adverse effect as a long-term dietary supplement. Daily administration of the fermented *Glycine max* (L.) extract to newborns or infants daily increases weight gain of the babies or infants. Similarly, increased weight gain can be achieved in infants of nursing mothers continuously taking the fermented *Glycine max* (L.) extract.

The fermented *Glycine max* (L.) extract can also enhance hemopoeitic and liver functions after a surgical operation as demonstrated through daily administration of 1 ml of the fermented *Glycine max* (L.) extract along with other therapeutic products to women undergoing operation after hospital admission except for the surgery day and several post-surgery days.

This invention will now be described with reference to the following non-limiting examples.

### Example 1

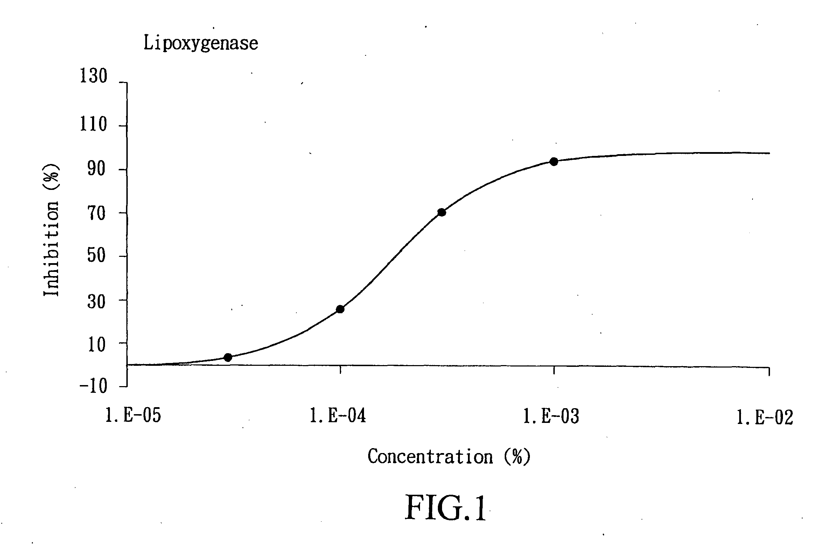

LOX-15 is the main metabolizing enzyme in arachidonate acid (AA) metabolism. One of the metabolic pathways of AA involves 15-lipoxygenase, LOX-15, which leads to the formation of HETE (hydroxyeicosatetraenoic acid). HETE has been reported to play an important role in cancer cell metastasis. HETE can induce protein kinase C activity to result in cancer cell metastasis. HETE is also a mitogenic factor, which results in angiogenesis of cancer cells. LOX-15 was isolated from rabbit reticulocytes. Linoleic acid was used as a substrate of LOX-15 with or without the fermented soybean extract. The amount of HETE formed was determined spectrophotometrically. The data show that the fermented soybean extract had an inhibitory effect on LOX-15 (see Figure 1). The result indicated that the fermented soybean extract inhibits angiogenesis and metastasis of cancer cells and induce apoptosis of cancer cells.

A further LOX-15 inhibition test was conducted according to the above-mentioned method to compare the inhibition efficacy of LOX-15 between the unfermented soybean and the fermented soybean extract. The results show that the inhibition efficacy of the fermented soybean extract was over four times of the unfermented soybean extract.

### Example 2

The fermented black soybean extract was tested in rabbit reticulocytes for the inhibitory effect on LOX-15. The test method refers to the method described in Analytical Biochemistry, 1992, 201, 375-380. The 1%, 0.5%, 0.1%, 0.05% and 0.01% (v/v) of black soybean extracts were used in incubating the reticulocytes at 4°C for 10 minutes. The incubation buffer is 0.05 M potassium phosphate buffer (pH5.9). The resulting 13-HPODE is quantified by Spectrophotometer.

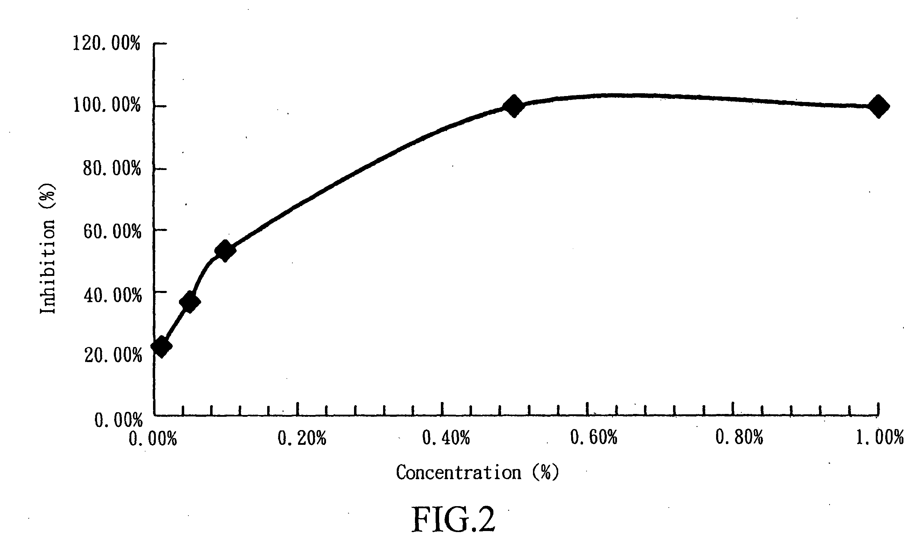

The relation of the concentration of the black soybean and its inhibition rate to LOX-15 was shown in Figure 2.

A further LOX-15 inhibition test was conducted according to the above-mentioned method to compare the inhibition efficacy of LOX-15 between the unfermented black soybean and the fermented black soybean extract. The LOX-15 inhibition rate of the unfermented black soybean was shown in below Table 1:

**Table 1**

| Unfermented Black Soybean (concentration, %) | LOX-15 Inhibition Rate (%) |
|---|---|
| 10 | 97.32 |
| 1.00 | 25.00 |

The LOX-15 inhibition rate of the fermented black soybean extract was shown in below Table 2:

**Table 2**

| Fermented black soybean extract (concentration, %) | LOX-15 Inhibition Rate (%) |
|---|---|
| 1.00 | 100.00 |
| 0.50 | 100.00 |
| 0.10 | 53.10 |
| 0.05 | 36.55 |
| 0.01 | 22.07 |

The results shown in the above tables show that the fermented black soybean extract has an unexpected superior efficacy in inhibiting LOX-15.

### Example 3

The fermented soybean extract functioned as an antioxidant and in the removal of free radicals. Several models published previously were used to study the antioxidant capacity of the fermented soybean extract, with Vitamin C and Trolox used as positive controls. The following methods were used for determining the antioxidant activity: (1) NBT method (2) H₂O₂ reduction method (3) DPPH reduction (4) TRAP reduction method (5) Conjugated diene (6) Lipid peroxidation (7) Chemiluminescence (see Figures 3, 4 and 5) in the presence of active oxygen. All results demonstrated that the fermented soybean extract has the highest antioxidant activity against unsaturated fatty acid and peroxidation compared with Vitamin C and Trolox.

Experiments demonstrated that the fermented soybean extract functions both as a antioxidant and free radical acceptor in the Okubo test system for chemiluminescence acceptor in the presence of active oxygen. The experiments were performed by measuring chemiluminescence in a liquid of hydrogen peroxide with or without acetaldehyde. Known antioxidants, e.g. gallic acid (see Figure 3(b)), EGC, tea and vitamin C (see Figures 4(a)-(c)) or the fermented soybean extract (see Figure 5) was added at 200 seconds. The data are shown in Figures 3-5. Figure 3(b) shows that chemiluminescence was increased at 200 seconds when gallic acid was added to a mixture of hydrogen peroxide and acetaldehyde. Figure 4 shows that when EGC, tea or vitamin C was added at 200 seconds, the chemiluminescence was increased when acetaldehyde was present. However, the chemiluminescence was also increased in the absence of acetaldehyde when vitamin C was added at 200 seconds (see Figure 4(c)) demonstrating that the anti-oxidant mechanism of vitamin C probably differs from that of EGC and tea. Figure 5 shows that, after the addition of the fermented soybean extract at 200 seconds, the chemiluminescence increased indicating that the fermented soybean extract was a powerful anti-oxidant. The anti-oxidant activity of the fermented soybean extract means that the fermented soybean extract can function in removing free radicals. With anti-oxidant and free radical removing functions, the fermented soybean extract is useful in promoting the general health of individuals or improving the health of subjects in need of health improvement because oxidative stresses, such as excessive presence of reactive oxygen species and lipid peroxidation, are known to be harmful to the body.

### Example 4

The anti-microbial activities of the fermented soybean extract were demonstrated by determining *with in vitro* methods. In the first experiment, *Salmonella typhimurium, Bacillus subtilis,* three strains (TMU-C74, TMU-D16 and TMU-E86) of *Helicobacter pylori* and vancomycin resistant *Enterococcus feacalis* were cultured in nutrient broth or BHI broth and transferred to Mueller Hinton agar plates or chocolate agar plates. The fermented soybean extract was put on a paper disk on the agar plate and the size of an inhibition zone was measured after incubation at 3 7° C. The data are shown in below Table 3:

**Table 3**

| Microbe | Fermented Soybean Extract | Inhibition Zone (mm) |
|---|---|---|
| *Salmonella typhimurium* | Undiluted | 11 |
| *Bacillus subtilis* | Undiluted | 14 |
| *H. pylori* TMU-C74 | Undiluted | 15 |
| *H pylori* TMU-D16 | Undiluted | 16 |
| *H. pylori* TMU-E86 | Undiluted | 15 |
| V.R. *E. feacalis* | Undiluted | 25 |
| V.R. *E. feacalis* | Diluted 50% | 15 |

In another experiment, the minimal inhibitory concentrations (MICs) of the fermented soybean extract were determined in *Salmonella typhimurium* (ATCC 14028), *Bacillus subtilis* (CRCC 10447), *Staphylococcus aureus* (ATCC 25923) and vancomycin resistant *Enterococcus feacalis.* Suspensions of these bacteria were adjusted to 3 x 10⁵ CFU/ml. The adjusted bacteria suspensions were added to a 96-well plate with or without various concentrations, i.e. 10%, 5%, 2.5%, 1.25%, 0.65%, or 0.32%, of the fermented soybean extract. The plate was incubated at 37° C for 15 hours. The MICs were determined after incubation and shown in below Table 4:

**Table 4**

| Microbe | MIC of Fermented Soybean Extract |
|---|---|
| *Salmonella typhimurium* | 2.5% |
| *Bacillus subtilis* | 2.5% |
| *Staphylococcus aureus* | 2.5% |
| *VR. Enterococcus feacalis* | 1.25% |

The fermented black soybean extract was also used in testing the anti-microbial activities. The MICs were determined and shown in below Table 5:

**Table 5**

| Microbe | MIC of Fermented Black Soybean Extract |
|---|---|
| *Pseudomonas aeruginosa* | 3.125% |
| *KP (Klebsiella pneumoniae)* | 3.125% |
| *M.R Staphylococcus aureus* | 1.563% |

### Example 5

The effects of the fermented soybean extract on immunity modulation were studied.

### (A) In vitro Studies:

### Spleen Cell Proliferation Assay (MTT Method)

Spleen cells were isolated from mice and put in a culture flask at 2 x 10⁶ cells/ml in a RPMI medium with or without one of several mitogens, i.e. lipopolysaccharide (LPS), concavalin A (Con A) and phytohemagglutinin (PHA). The spleen cell cultures were incubated overnight for MTT assay

A sub-optimal concentration of 5 ug/ml of LPS combined with the fermented soybean extract at 1%, 0.5%, 0.1%, 0.05% or 0.01%, had no effect on spleen cell proliferation, especially for B cells. A concentration of 5 ug/ml of PHA combined with 0.05% of the fermented soybean extract increased the spleen cell number, especially for T cells, which was 2.32 fold of the spleen cell number obtained with PHA alone. According to this result, the fermented soybean extract has an effect on T and B cell interaction in immunity modulation. A concentration of 5 ug/ml of Con A combined with 0.05% of the fermented soybean extract produced a spleen cell number which was about 20% less than the spleen cell number obtained with Con A alone. According to this result, the fermented soybean extract could play a role in anti-inflammation reactions.

### Macrophage Activity Assay.

Balb/c mice were injected with thiogllate. Three to four days after the injection, macrophages were isolated from the peritoneal cavity of the mouse and incubated with or without the fermented soybean extract at 37° C for 30 minutes. *E. coli* cells conjugated with a fluorescence probe were added to the macrophage suspension and incubated at 37° C for 2 hours. A phagocytosis assay was conducted with flow cytometry. The data showed that the fermented soybean extract at 0.05% enhanced the phagocytosis activity of the macrophage by about 71 % compared with macrophages not treated with the fermented soybean extract.

### (B) In vivo Studies

Male ICR albino mice were injected intraperitoneally with vehicle, 0.8 ml of 1% of the fermented soybean extract per mouse, 0.8 ml of 0.1% of the fermented soybean extract per mouse, Levamisole at 30 mg/kg, or azimexone at 100 mg/kg. One hour after the intraperitoneal injection, *Candida albican* (ATCC 10231) was injected intravenously into the mouse at 1.5 to 2 x 10⁷ CFU per mouse. The mortality of the mouse was determined daily for 10 days (see Table 6).

As shown in Table 6, the fermented soybean extract reduced the mortality of *Candida albican* in the mouse. The mortality reductive effect of the fermented soybean extract was more pronounced than that of levamisole.

Male ICR albino mice were pretreated with cyclophosphamide at 30 mg/kg on days 5, 3 and 1 before injected intravenously with *Candida albican.* On days 6, 4 and 2 before the intravenous injection of *Candida albican,* the mouse was treated with vehicle, 0.1% of the fermented soybean extract, 1% of the fermented soybean extract or azimexone at 100 mg/kg. The mortality of the mouse was determined daily for 10 days (see Table 7). As shown in Table 7, with cyclophosphamide pretreatment, the fermented soybean extract reduced the mortality of *Candida albican* in the mouse. The mortality reductive effect of the fermented soybean extract was comparable to that of azimexone.

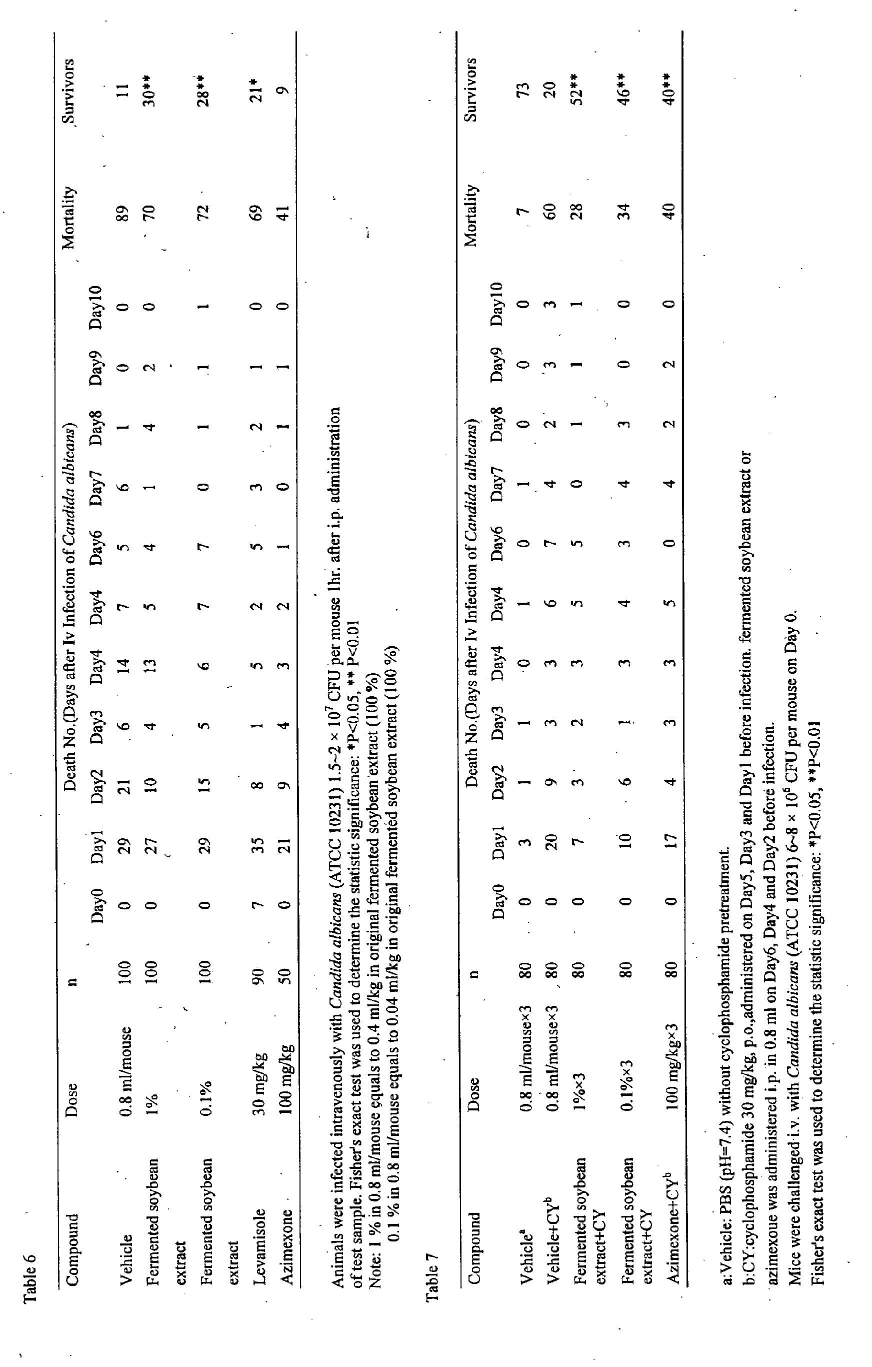

### Example 6

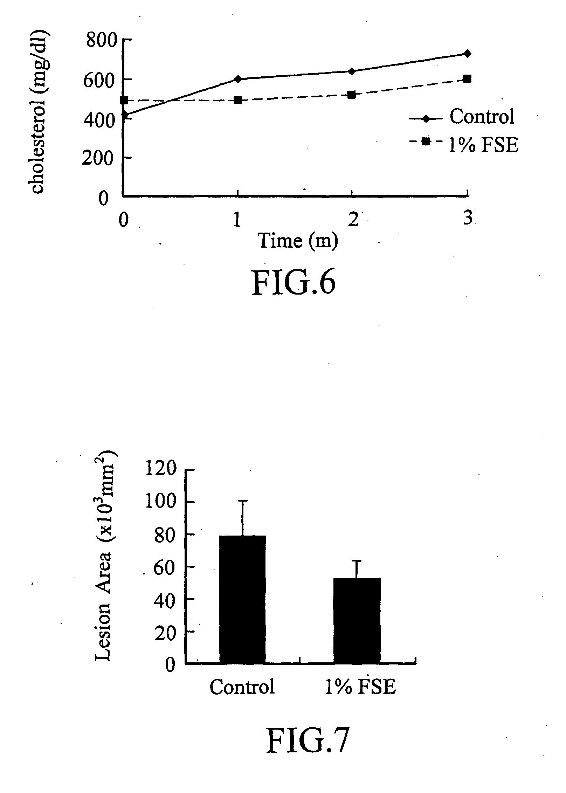

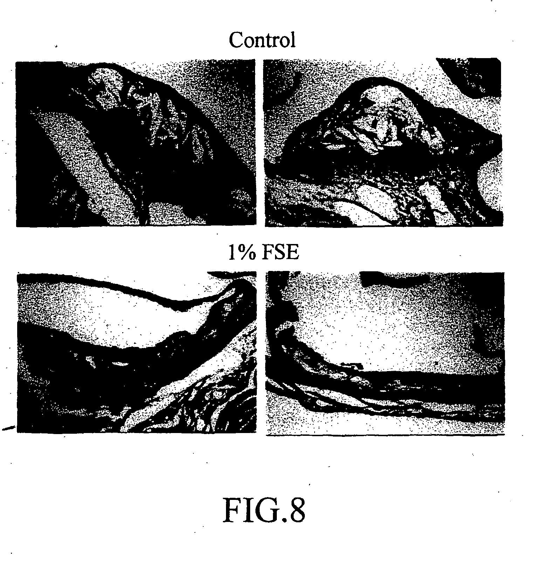

The fermented soybean extract was tested in apoE-deficient mice (with spontaneously hypercholesterolemia and atherosclerosis lesions similar to humans) for evaluating the effect in treating cardiovascular diseases. ApoE-deficient mice were fed with 10 µl/g of fermented soybean extract product (15% in concentration) for 3 months, and then the level of plasma cholesterol and the extent of atherosclerosis lesion were measured. It is found that the treatment with the fermented soybean extract product of the present invention does not significantly affect the level of plasma cholesterol, but the lesion of arherosclerosis in mice receiving the fermented soybean extract product of the present invention is substantially reduced in comparison with the control (see Figures 6, 7 and 8). According to the results, the efficacy of treating cardiovascular disease such as arteriosclerosis of the fermented soybean extract product of the present invention is achieved by the mechanism of inhibiting 15-lipoxygenase, rather than lowering the level of plasma cholesterol.

### References

Adlercreuz, H. et al., Evaluation nutrition, intestinal microflora and prevention of cancer: a hypothesis, Proc. Soc. Exp. Biol. Med., 217:241-246 (1998).

Breimer LH. Ionizing radiation-induced mutagenesis, Br J Cancer, 57:6-18 (1998).

Briehl, M. M. et al., Modulation of the antioxidant defense as a factor in apoptosis, Cell Death Differ., 3:63-70 (1996)

Chemoprevention Working Group to the American Association for Cancer Research, Cancer Res. 59:4743-4758 (1999).

Cohen, L. A.et al., Effect of intact and isoflavone-depleted soybean protein on NMU-induced rat mammary tumorigenesis, Carcinogenesis, 2: 929-935 (2000).

Dwyer, J.T. et al., Tofu and soybean drinks contains phytoestrogenes, J. Am. Diet Assoc., 94: 739-743 (1994).

Ghibelli, L. et al., Rescue of cells from apoptosis by inhibition of active GSH extrusion, FASEB J., 12: 479-486 (1998).

Greenwald, P. et al., Chemoprevention, CA-Cancer J. Clin., 45:31-49 (1995). Hong, W. K. et al., Recent advances in chemoprevention of cancers, Science, 278:1073-1077 (1993).

Hutchins, A. M. et al., Urinary isoflavoneoid phytoestrogen and lignan excretion after consumption of fermented and unfermented soybean products, J. Am. Diet Assoc., 95:545-551 (1995).

Ikeda, Y. et al., The molecular basis of brain injury and brain edema: the role of oxygen free radicals, Neurosurgery, 27:1-11 (1990).

Keisari, Y. et al., A simple colorimetric method for the measurement of hydrogen peroxide produced by cells in culture, J. Immunol Methods., 38:161-170 (1980).

Kelloff, G. J., Approaches to the development and marketing approval of drugs that prevent cancer, Cancer Epidermiol. Biomarkers Pre., 4:1-10 (1995).

Kontos HA et al., Oxygen radicals in brain injury, CNS Trauma, 3:257-63 (1986).

Messina, M. et al., Soybean intake and cancer risk: a review of the in vitro and in vivo data, Nutr. Cancer, 21:113-131 (1994).

Nout, M. J. R. et al., Recent development in temphe research, J. Appl. Bacteriol., 69:609-633 (1990).
Plamer, H. J.et al., Reactive oxygen species and antioxidants in signal transduction and gene expression, Nutr. Rev, 55: 353-361 (1997).

Robak J. et al., Flavonoids are scavengers of superoxide anions, Biochemical Pharmacology, 37(5):837-41 (1988).

Shao, Z. M. et al., Genistein exerts mutiple suppressive effects on human breast carcinoma cells, Cancer Res., 58:4851-4857 (1998).

Steinberg D. et al, Beyond cholesterol: modifications of low-density lipoprotein that increase its atherogenicity, N Engl J Med, 320:915-24 (1989).

Toshiki, Y. et al., Mechanism of catechin chemiluminescence in the presence of active oxygen, J. Biolumin. Chemilumin., 11:131-136 (1996).

Wang, H. et al., Isoflavone content of commercial soys foods, J. Agric. Food Chem., 42:1666-1673 (1994).

## Claims

1. Use of a fermented *Glycine max* (L.) extract for the production of a composition for use in the inhibition of 15-lipoxygenase for treating or reducing the risk of cardiovascular disease in a subject, wherein the fermented *Glycine max* (L.) extract is prepared by fermenting an aqueous *Glycine max* (L.) extract with at least one lactic acid bacteria together with at least one yeast.

2. The use of claim 1, wherein the *Glycine max* (L.) is black soybean.

3. The use of claim 1 or 2, wherein the lactic acid bacteria is a *Lactobacillus* species.

4. The use of any one of the preceding claims, wherein the yeast is a *Saccharomyces* species.

5. The use of any one of the preceding claims, wherein said cardiovascular disease is atherosclerosis.

6. The use of any one of the preceding claims, wherein the composition is a pharmaceutical composition or a food composition.
